Europäisches Patentamt

European Patent Office

Office européen des brevets

(11) Publication number: **0 256 192**
**A1**

## EUROPEAN PATENT APPLICATION

(21) Application number: **86306468.9**

(22) Date of filing: **20.08.86**

(51) Int. Cl.³: **C 08 J 9/24**
**A 61 L 15/00**

(43) Date of publication of application:
**24.02.88 Bulletin 88/8**

(84) Designated Contracting States:
**BE DE FR GB IT NL**

(71) Applicant: **ETHYL CORPORATION**
**451 Florida Boulevard**
**Baton Rouge, LA 70801(US)**

(72) Inventor: **Goodrum, Richard Warren**
**11821 Bollingbrook Drive**
**Richmond Virginia 23236(US)**

(74) Representative: **Bizley, Richard Edward et al,**
**BOULT, WADE & TENNANT 27 Furnival Street**
**London EC4A 1PQ(GB)**

(54) Porous film and absorptive structure.

(57) An absorptive structure for absorbing and containing fluids from a source exterior of said structure comprising a topsheet (11), an absorbent element (12) and a back sheet (13) wherein said topsheet is a liquid permeable material formed from particles of polymeric materials partially fused together to form a continuous sheet and has a multiplicity of openings therein of a predetermined size and shape so as to direct fluid flow into the absorbent element and inhibit fluid flow from the absorbent element through the topsheet, and said back sheet is impervious to liquids.

FIG. 2.

- 1 -

## POROUS FILM AND ABSORPTIVE STRUCTURE

The present invention is in the field of plastic films or sheets and more particularly relates to porous or liquid permeable thermoplastic films.

Porous or perforated thermoplastic films have many useful applications. Such films are useful in gardening and farming to prevent the growth of grass or weeds while permitting moisture to be transmitted through the film to the soil beneath. They are used for making disposable diapers and other various absorbent structures and for packaging of foods and other materials.

The invention also relates to absorptive structures made from the porous film, such as diapers, sanitary napkins, bed pads, incontinent pads, towels, bandages and the like. The invention particularly relates to porous film used as topsheets for such structures.

The invention especially relates to an improved porous film or topsheet which allows fluid to pass to the interior of the absorptive device but which inhibits the reverse flow of the fluid. In general, the topsheet is the portion of an absorptive device which covers one face of the absorbent element of the absorptive structure and which in some applications contacts the skin of a person using the absorptive device.

Particularly useful absorptive devices are articles of manufacture designed to receive and retain fluid discharges from the body within an absorbent element of the absorptive device. Absorptive devices such as sanitary napkins, catamenial tampons, bed pads, incontinent pads, towels, bandages and the like are well known articles of commerce. In recent times, single use disposable absorptive devices have significantly replaced permanent absorptive devices which were designed to be laundered and reused. While the improved absorptive structure of this invention can be used with reusable absorptive devices, it finds great utility when used with disposable absorptive devices.

Perforated thermoplastic films of polyethylene, polypropylene, polybutene-1, polyvinyl chloride, and other flexible thermoplastics normally extruded into such films or thin sheets have been made by various methods. One method is to extrude the thermoplastic material, e.g., polyethylene, from a conventional slot extrusion die onto a continuously moving, smooth, cooled casting surface, e.g., a chill roll. A pattern may be applied to the chill roll and the film pressed to the roll while in the amorphous or molten stage by press rolls.

An example of a method and apparatus for producing film according to the foregoing slot die-chill cast roll technique is shown in U.S. 3,374,303.

Another technique used for making porous plastic film has been the utilization of a heated engraved embossing roll in conjunction with a backup roll. The preformed strip of thermoplastic film normally at room temperature, is passed between the nip of a heated engraved roll and a backup roll and is embossed by being heated while in contact with the heated, engraved roller. The resultant embossed film usually has a very shallow and poorly defined pattern. An example of an apparatus and process for carrying out a process of this type is shown in U.S. 3,176,058.

Still another process for making porous thermoplastic film has been to pass the film over a heated roll or a series of heated rollers in order to heat the film to a softened state and then to contact the film with an embossing roller and to press the film against the embossing roller by a backup roller. Normally, the embossing roller and the backup rollers are cooled in order to freeze the pattern into the film so that it may be immediately wound up into rolls, if desired. An apparatus and process for preparing an embossed film according to the foregoing is shown in U.S. 3,246,365.

A more recent process for making such plastic material is shown in U.S. 3,950,480, wherein the film is heated by a non-direct contact heat source to raise the temperature of the film above its softening point and the film is then immediately fed between adjacent, counterrotating rollers, and thereby embossed.

A method for perforating thermoplastic sheet or film is disclosed in U.S. 3,054,148, issued to Zimmerli. The Zimmerli patent discloses a stationary drum having a molding element screen mounted around the outer surface of the drum which is adapted to rotate freely thereon. A vacuum chamber is employed beneath the screen or molding element to create a pressure differential between the respective surfaces of the thermoplastic sheet to cause the plasticized sheet to flow into the perforations provided in the molding element and thereby cause a series of holes to be formed in the sheet.

U.S. 4,155,693 and U.S. 4,157,237 illustrate types of screens or molding elements.

U.S. 4,252,516 and U.S. 4,317,792 disclose apparatus and method, respectively, for manufacturing thermoplastic sheet having elliptical holes.

Disposable absorptive devices comprising an absorbent pad covered with a topsheet which contacts the body are well known. Covering the outer portion of the absorptive device with a fluid-impermeable backsheet to prevent absorbed fluids from leaking out of the absorptive device and soiling clothing, bed clothes, etc. is equally well known. The absorbent pad component of disposable absorptive devices can comprise well known materials such as creped cellulose wadding, airlaid felt or the like. The liquid impermeable backsheet can comprise any of various materials well known in the art such as polyethylene film.

One of the principle disadvantages of conventional absorptive devices is the maceration of the skin caused by prolonged contact with absorbed fluids. One especially common manifestation of this maceration is diaper rash generally occurring about the base of the trunk of infants. In order to minimize the effect of prolonged liquid contact with the skin, absorptive devices such as diapers have been produced with the body contacting topsheet designed to exhibit a greater or lesser degree of surface dryness. For example, U.S. Patent No. 3,327,625 issued to Johnson on March 1, 1966, teaches that any hydrophobic material in the crotch area of the diaper will cause moisture to wick away from the skin of an infant wearer and thereby provide a substantially dry surface in contact with the infant's skin. U.S. Patent No. Re. 26,151 issued to Duncan et al. on January 31, 1967 teaches the use of porous, hydrophobic, nonwoven fabrics as topsheets. U.S. Patent No. 2,916,037 issued to Hansen on December 8, 1959, is a further example of the use of a nonwoven topsheet.

U.S. Patent 3,814,101 issued to Kozak on June 4, 1974 illustrates still another type of disposable absorbent article. Such patent discloses a topsheet of non-fibrous hydrophobic film which has a plurality of valvular openings or slits therein and a system of depressed areas disposed across the surface of the topsheet. The openings permit the flow of liquid in one direction of the absorbent but reduce the flow of the liquid in the opposite direction.

U.S. Patent No. 3,989,867 which issued to Sisson on November 2, 1976, describes a breathable liquid impervious backsheet containing apertured bosses. The apertures therein, in order to maintain the liquid impervious character of the backsheet, are smaller in diameter than the capillaries of U.S. 3,929,135 hereinafter described.

U.S. Patent 3,929,135 issued to Thompson on December 30, 1975, relates to absorptive devices utilizing a topsheet having tapered capillaries of critical diameters and tapers which allow fluid to pass into the interior of an absorptive device and which inhibit the reverse flow of such fluid.

The present invention relates to a unique porous sheet of thermoplastic material, a perforated porous sheet or film and an absorptive device made from such sheet or film. The porous film comprises a liquid permeable material formed from particles of polymeric materials partially fused together to form a continuous sheet. The sheet may also have a multiplicity of additional openings or perforations therein of a predetermined size and shape so as to direct the flow of fluids in one direction or into an absorbent element and to inhibit the flow of fluids in the other direction or from the absorbent element through the sheet. The perforations or additional holes may be added to the sheet by slitting, perforating, or any other suitable means.

The sheet of the instant invention is an improvement over prior art sheets in that it enhances the free transfer of fluids into an absorbent substrate and more effectively inhibits the reverse flow of the fluids from the substrate. The sheet or film closely resembles and feels like very soft cloth.

It is desired to provide a porous plastic film constructed of partially heat fused fine polymeric particles.

It is further desired to provide a porous plastic film which has an additional multiplicity of small perforations or openings therein.

It is also desired to provide an absorptive structure for absorptive devices which enhances the free transfer of fluids from an exterior source into a substrate absorbent element while effectively inhibiting the reverse flow of fluids from the absorbent element.

It is still further desired to provide a sheet or topsheet for absorptive devices or other use which feels smooth to the human touch and has the feeling of cloth.

The construction designed to carry out the invention will be hereinafter described, together with the features thereof.

The invention will be more readily understood from a reading of the following specification and by reference to the accompanying drawings forming a part thereof, wherein an example of the invention is shown and wherein:

FIG. 1 is a perspective representation of an absorptive structure of the invention with a portion of its components cut away.

FIG. 2 is an enlarged cross-section in elevation of the absorptive structure taken along line 2-2 of FIG. 1;

FIG. 3 is an enlarged cross-section of a porous sheet or film of the invention and the topsheet of FIG. 2.

The absorptive structure of the invention is generally referred to by the reference numeral 10. The novel porous film or topsheet of this invention is shown at 11. The other major components of the absorptive structure 10 are the absorbent element or pad 12 and the backsheet sheet 13. In the drawings, like characters of references designate like parts throughout the several views.

In general, the side flaps 14 of the backsheet 13 are folded so as to cover the edges of the absorbent pad 12 and topsheet 11. Such arrangement completely seals in the absorbent pad 12. Any other arrangement for sealing the edges of the absorbent structure may be used without departing from the scope of the invention.

The structure of the porous film or topsheet 11 comprises a plurality of particles 20 of materials partially fused together at 21 to form a continuous sheet. The continuity of the particles is further interrupted by a multiplicity of openings 22 which may be in the shape of slits, dimples, funnels, tapered capillaries, cylinders or other geometric and asymmetric shapes and may be varied in size and frequency to suit the particular viscosity, density, mass and flow rates of the fluid to be absorbed. U.S. 3,929,135 and U.S. 3,814,101 illustrate openings or holes of a suitable size and shape.

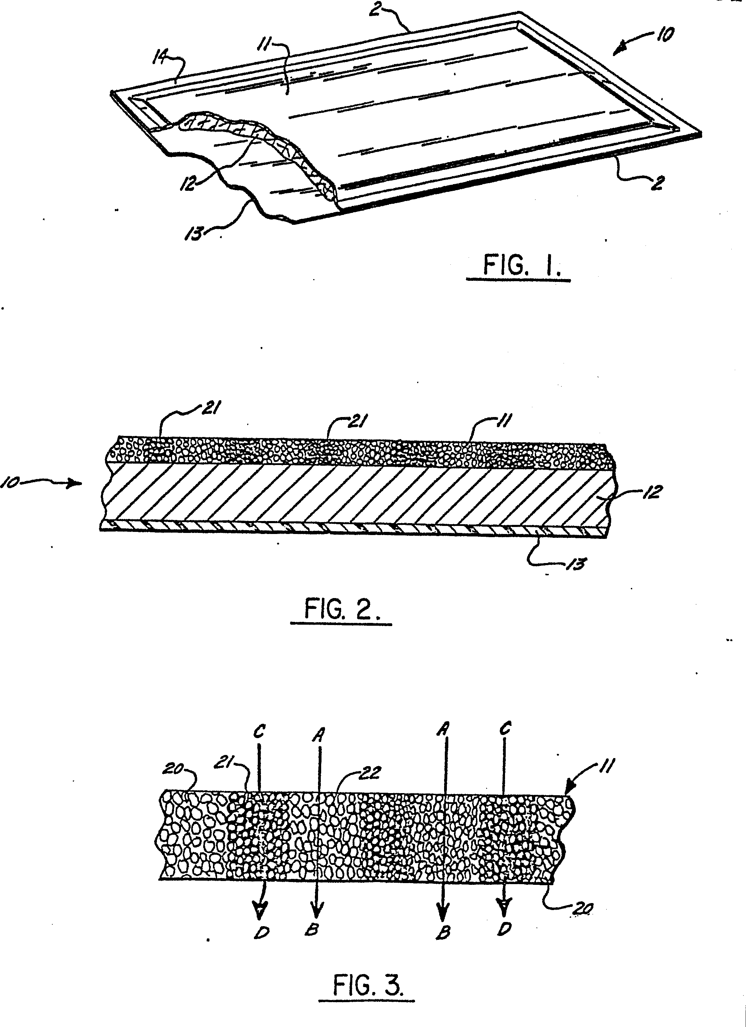

It can readily be seen in FIG. 3, that fluids flow directly through the porous film or topsheet from A to B and also may flow indirectly from C to D.

Aesthetically, the structure of the porous film or topsheet closely resembles and feels like very soft cloth. Ideal particle sizes are those which are small enough to feel smooth to a human touch. Particles from 0.003 inches to 0.004 inches (0.07mm to 0.10mm) in diameter are suitable Sizes as small as 1 or 2 microns and as large as 2000 microns are also suitable.

It is not necessary, of course, that the particles all be of the same size. A mixture of particles of various sizes within the desired range of sizes is quite suitable. In fact, such particles are normally obtained as mixtures of various sizes of particles. Materials which have been found to meet the specifications and which are generally supplied in particle form are polymeric materials such as the linear low density polyethylenes, high density polyethylenes, polypropylene and polyvinylchloride (PVC).

Fusing sintered particles of all sorts of materials that melt with heat and pressure are well known and old in the art. The porous film of the invention is made by the application of such art in a continuous process. Although the particles are relatively small, heat and pressure are applied thereto only in an amount sufficient to provide a desired fusion which in turn provides a desired degree of porosity. The particles themselves are not permeable. It is only the spaces provided from partially fusing the particles which provides permeability to the sheet. Perforating the finished sheet provides additional permeability, if such is desired.

Additional strength may be imparted to the porous film or sheet by post orienting in one or two directions by the use of a tentering frame which also increases the rate of fluid flow through the sheet.

The manufacture of the porous sheet may be by any suitable method such as dispersion of the polymeric particles on a moving belt followed by heating to a temperature appropriate for the desired degree of fusion of the polymeric particles thereby forming the porous sheet. Thereafter, the sheet is passed through a set of pressure rollers, cooled and then stripped from the belt, the stripped sheet being wound into a roll. A variation of such process provides for preheated particles being distributed into the nip of an embossing set of rollers wherein the particles under pressure from the embossed rolls are fused and simultaneously embossed with a pattern of holes designed to increase the porosity of the sheet. Selection of the particular embossed pattern along with variations in particle size may be combined over a wide range of shapes, frequency and sizes to impart not only increased flowability through the topsheet but improved functionality and aesthetically pleasing and comfortable surfaces. Post orientation may further enhance such properties while improving the strength characteristics of the sheet.

Excluding the area of holes formed by the voids between partially fused particles, a porous sheet with total projected open area of from less than 1% to 64% of the total area, resulting from the geometrically or asymmetrically shaped holes added to the sheet by embossing, perforating, piercing, vacuum forming or other processes used to impart holes and prior to post orienting satisfies the requirements of the practical uses of the invention and also satisfies the requirements of serviceability for strength applications. Beginning with such percentages of open areas, post orienting can at least double the stated projected areas. The maximum would represent a seven-fold stretch of the base sheet in both transverse and machine direction. Such amount has been found to enhance the desired properties from the olefin polymers found to be most suitable in such applications.

A projected open area in the porous sheet of about 5% represents holes, whether geometric in design or asymmetric. The holes or openings have a mean diameter of 0.005 inches (0.13 mm) spaced an average of 0.020 inches (0.51 mm) apart. A projected open area of about 20% may be obtained by holes with a mean diameter of either 0.005 inches (0.13 mm), 0.010 inches (0.26 mm), 0.020 inches (0.51 mm), 0.05 inches (1.3 mm) or 0.10 inches (2.5 mm) and with respective spacing of 0.010 inches (0.26 mm), 0.20 inches (5 mm), 0.040 inches (1 mm), 0.10 inches 2.5 mm) and 0.20 inches (5 mm). A practical maximum projected open area for a non-oriented, porous sheet is about 70% due to the fragility of the connecting pieces between holes. Projected area percentages are based on square inch sized areas.

The topsheet of the invention is constructed of finely divided particles which range from a size of 0.003 inches to a size of 0.004 inches (0.07mm to 0.10mm) or from 1 to 2000 microns. These finely divided particles are partially fused together by heat to provide a thin porous sheet having a thickness of from 0.0005 inches to 0.25 inches (0.01mm to 6.35mm).

If the porous sheets hereindescribed are post oriented through a tentering apparatus to high degree, projected areas must be based on units larger than square inches, e.g., square feet.

The thickness of the non-oriented porous sheet varies from 0.0005 inches to 0.250 inches (0.01mm to 6.35mm) depending upon the particle size and the particular embossing pattern. Orientation also reduces these thicknesses in direct proportion to the degree of orientation.

According to a further aspect of the present invention there is provided the use of a sheet-material comprising particles of polyolefin or polyvinylchloride polymeric materials partially fused together by heat, said particles ranging in size, from about one micron to about 2000 microns, and said sheet having a thickness of about 0.0005 inches (0.01 mm) to about 0.25 inches (6.35 mm) as a liquid permeable barrier.

The foregoing disclosure and description of the invention is illustrative and explanatory thereof and various changes in the illustrated structure may be made within the scope of the invention.

CLAIMS

1.  A liquid permeable sheet material having the feel and resemblance of soft cloth comprising particles of polyolefin or polyvinylchloride polymeric materials partially fused together by heat so as to provide a desired amount of liquid permeability, said particles ranging in size from about one micron to about 2000 microns, and said sheet having a thickness of about 0.0005 inches (0.01 mm) to about 0.25 inches (6.35 mm).

2.  A sheet as claimed in claim 1, in which said particles of polymeric materials are post oriented to improve strength of the sheet.

3.  A sheet as claimed in claim 1 or claim 2, in which said particles of polymeric materials are of a size ranging from about 0.003 inches (0.07 mm) to about 0.004 inches (0.10 mm).

4.  A sheet as claimed in any one of claims 1 to 3 in which said particles of polyolefin polymeric materials are polyethylene or polypropylene.

5.  A sheet as claimed in any one of claims 1 to 4, in which said sheet has a multiplicity of additional openings therein of a predetermined size and shape, said openings being so constructed as to direct liquids therethrough in one direction and to inhibit the flow of liquids therethrough in the other direction.

6.  A sheet as claimed in claim 5, in which in a given projected open area of the sheet, about five percent or more of the area represents said additional openings.

7. A sheet as claimed in claim 5 or claim 6 in which said additional openings have a mean diameter of about 0.005 inch (0.13 mm) and are spaced apart an average of about 0.02 inch (0.51 mm).

8. An absorptive structure for absorbing and containing fluids from a source exterior of said structure comprising a topsheet, an absorbent element and a back sheet, in which said topsheet is a liquid permeable material formed from particles of polyolefin or polyvinylchloride polymeric materials partially fused together by heat said particles of a size ranging from about one micron to 2000 microns, said topsheet having a thickness of about 0.0005 inches (0.01 mm) to about 0.25 inches (6.35 mm).

9. An absorptive structure as claimed in claim 8, wherein the topsheet is further defined by the features of any one of claims 2 to 7.

10. The use of a sheet-material comprising particles of polyolefin or polyvinylchloride polymeric materials partially fused together by heat, said particles ranging in size, from about one micron to about 2000 microns, and said sheet having a thickness of about 0.0005 inches (0.01 mm) to about 0.25 inches (6.35 mm) as a liquid permeable barrier.

1/1

0256192

FIG. 1.

FIG. 2.

FIG. 3.

## EUROPEAN SEARCH REPORT

**European Patent Office**

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 048 537 (D.B. PALL et al.) <br> * Claims 1,2,7,8; column 3, line 28 - column 4, line 16; examples 1-7; column 6, line 75 - column 7, line 3 * | 1,3,4 | C 08 J 9/24 <br> A 61 L 15/00 |
| | --- | | |
| X | CH-A- 365 871 <br> (AKKUMULATORENFABRIK Dr. LEOPOLD JUNGFER) <br> * Claims, subclaims; page 1, paragraph 4 * | 1,2,4 | |
| | --- | | |
| X | US-A-2 371 868 (H. BERG et al.) <br> * Claims; page 2, right-hand column, lines 46-51; page 3, left-hand column, lines 5-40, right-hand column, lines 3-17; page 4, left-hand column, lines 41-42; page 5, left-hand column, line 43 - right-hand column, line 12 * | 1,8,9 | **TECHNICAL FIELDS SEARCHED (Int. Cl.4)** <br><br> C 08 J <br> A 61 L |
| | --- | | |
| X | AT-A- 294 324 (BUNZL et al.) <br><br> * Claims 1,3; page 2, paragraph 1, example 2 * | 1,3,4, 8,9 | |
| | --- | | |
| A,D | US-A-3 814 101 (T.F. KOZAK) <br> * Claims 1-10 * | 5,6,7 | |
| | ---     -/- | | |

The present search report has been drawn up for all claims

| Place of search | Date of completion of the search | Examiner |
|---|---|---|
| THE HAGUE | 22-04-1987 | HALLEMEESCH A.D. |

| | DOCUMENTS CONSIDERED TO BE RELEVANT | | Page 2 |
|---|---|---|---|
| Category | Citation of document with indication, where appropriate, of relevant passages | Relevant to claim | CLASSIFICATION OF THE APPLICATION (Int. Cl.4) |
| A,D | US-A-3 929 135 (H.A. THOMPSON) <br> * Claims 1-8 * <br><br> ----- | 5,6,7 | |
| | | | **TECHNICAL FIELDS SEARCHED (Int. Cl.4)** |
| | The present search report has been drawn up for all claims | | |

| Place of search | Date of completion of the search | Examiner |
|---|---|---|
| THE HAGUE | 22-04-1987 | HALLEMEESCH A.D. |